(19) **Europäisches Patentamt / European Patent Office / Office européen des brevets**

(11) **EP 3 972 567 B1**

(12) # EUROPEAN PATENT SPECIFICATION

(45) Date of publication and mention
of the grant of the patent:
**13.08.2025   Bulletin 2025/33**

(21) Application number: **20727606.4**

(22) Date of filing: **20.05.2020**

(51) International Patent Classification (IPC):
**A61K 9/20** (2006.01)

(52) Cooperative Patent Classification (CPC):
**A61K 9/2095; A61K 9/2018; A61K 9/2027;
A61K 9/2054; A61K 9/2059**

(86) International application number:
**PCT/EP2020/064035**

(87) International publication number:
**WO 2020/234335 (26.11.2020 Gazette 2020/48)**

(54) **LACTOSE POWDER BED THREE DIMENSIONAL PRINTING**

DREIDIMENSIONALES LAKTOSEPULVERBETTDRUCKEN

IMPRESSION 3D SUR LIT DE POUDRE DE LACTOSE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.05.2019   EP 19175639**

(43) Date of publication of application:
**30.03.2022   Bulletin 2022/13**

(73) Proprietor: **DFE Pharma GmbH & Co. KG
47574 Goch (DE)**

(72) Inventor: **VAN DEN HEUVEL, Korinde
6708 WH Wageningen (NL)**

(74) Representative: **FrieslandCampina IP Department
Bronland 20
6708 WH Wageningen (NL)**

(56) References cited:
**US-A1- 2002 106 412     US-A1- 2011 187 015
US-A1- 2012 207 929**

• **MOOLCHANDANI VIKAS ET AL:
"Characterization and selection of suitable
grades of lactose as functional fillers for capsule
filling: part 1", JOURNAL DRUG DEVELOPMENT
AND INDUSTRIAL PHARMACY, vol. 41, no. 9, 2
September 2015 (2015-09-02), US, pages 1452 -
1463, XP093091239, ISSN: 0363-9045, DOI:
10.3109/03639045.2014.957703**

**Description**

FIELD OF THE INVENTION

The invention pertains to the production of pharmaceutical compositions by three-dimensional printing. Particularly, the invention pertains to tablets produced by 3D printing onto suitable lactose particles.

BACKGROUND TO THE INVENTION

Three-dimensional (3D) printing is a relatively new area of technology for making solid pharmaceutical dosage forms. It has become of increased interested since, in 2015, the FDA approved the first 3D printed tablet (Spritam® for the treatment of epilepsy). The 3D printing technology used therein is an inkjet-based 3D printing process, known as binder jetting. This process works in a layer-by-layer fashion where droplets of a liquid comprising a binder are selectively dispensed through a print head onto a thin layer of powder. The liquid can be a solution of a drug in a solvent, mixed with excipients to form a liquid paste. The liquid can also be without a drug, with a drug being provided blended with the powder. The aforementioned deposition of liquid drops onto a powder bed generates a two-dimensional layered print pattern, whereby the powder particles become bound together. The powder bed is then lowered, and a new layer of powder is spread, generally using a counter-rotating roller. Subsequently, droplets of the binder liquid are dispensed over the new layer of powder. The foregoing process is repeated until the desired 3D shape is obtained.

In the pharmaceutical field, the 3D printing process has potential advantages in several areas. For example, the use of 3D printing for the preparation of clinical phase 1 and 2 trial material potentially allows saving development time. 3D printing also opens the door for personalized medicine, as it will be easier to produce on a small scale tablets containing personalized doses or combinations of doses. In addition, it would be possible to achieve problematic formulations such as relatively low or relatively high dosed drug substances, poorly soluble drug substances, and various challenging controlled release mechanism.

The 3D printing of pharmaceutical formulations is considerably different from traditional formulation techniques. Accordingly, different requirements will hold for the excipients to be used, and existing knowledge in the area of pharmaceutical formulation technology cannot be directly transferred to the area of 3D-printing. The choices of raw materials for binder jetting, however, are limited and for regulatory reasons it is not preferred to look beyond excipients that are known to be pharmaceutically acceptable. Further, whilst difficulties may have to be overcome in any event, these are more challenging, if - as generally desired - an aqueous binder jetting liquid is used rather than an organic solvent such as an alcohol.

US 2002/0106412 relates to a method for controlling the migration of a binder liquid in a bulk powder. The bulk powder may be deposited in a powder bed that contains at least two different substances, each in powder form. One substance gives the printed part its bulk properties, forms most of the powder, and preferably is either insoluble or not significantly soluble in the binder liquid. The other powder substance is a migration control substance.

US 2011/0187015 related to an uniaxially compressed dosage form manufactured by three-dimensional printing that preserves the predetermined internal architecture of the dosage form while producing an improved surface finish.

The skilled person is clearly faced with a challenge to find excipients that are suitable for pharmaceutical 3D printing. For example, not all excipients that would technically be suitable are known as pharmaceutically acceptable. Some (polymeric) excipients yield a dense structure, limiting their applicability to controlled release, rather than being suitable for immediate release formulations.

Accordingly, it is desired in the art to find excipients that are of a known pharmaceutically acceptability from conventional tabletting techniques, and that have the right properties for the specific, entirely different, technique of 3D printing by binder jetting. Preferably, it is desired to provide excipients that are suitable for 3D printing of pharmaceutical formulations when using an aqueous binder jetting liquid.

Indicators for suitability include a proper wetting of the excipient particles, adequate consolidation of the printed structure, and the avoidance of bleeding of liquid from the formulation.

SUMMARY OF THE INVENTION

In order to better address the aforementioned desires, the invention presents, in one aspect, the use of lactose particles in the production of pharmaceutical formulations by three-dimensional (3D) printing, said printing comprising the repeated deposition of liquid drops onto superimposed layers of a powder bed, wherein the powder bed comprises lactose particles having a particle size distribution characterized by a D10 of at least 6 $\mu$m and a D9 0 value below 200 $\mu$m; and wherein the particle size distribution is characterized by a span of 2.0 or lower, and wherein particle size determinations are performed using a Helos Sympatec Particle Size Distribution analyzer, as further disclosed in Example 1 and wherein the span is defined as Span = (D90 - D10)/ D50.

In another aspect, the invention provides a process of preparing a solid pharmaceutical formulation by three-dimensional (3D) printing, comprising providing a first layer of a powder, depositing a pattern of binder liquid onto said first layer so as to provide a bound layer of powder, applying a second layer of the powder onto the first layer, depositing a pattern of binder liquid onto said second layer so as to provide a set of two bound layers of powder, continuing to provide further layers of powder each followed by further deposition of binder liquid, so as to provide a printed three-dimensional solid pharmaceutical formulation, wherein the powder comprises lactose particles having a particle size distribution characterized by a D10 of at least 6 $\mu$m and a D90 value below 200 $\mu$m; and wherein the particle size distribution is characterized by a span of 2.0 or lower, and wherein particle size determinations are performed using a Helos Sympatec Particle Size Distribution analyzer, as further disclosed in Example 1 and wherein the span is defined as Span = (D90 - D10)/ D50.

In a still further aspect, the invention presents a kit for generating a powder bed suitable in the production of pharmaceutical formulations by 3D printing, the kit comprising lactose particles and binder particles, wherein the lactose particles have a particle size distribution characterized by a D10 of at least 6 $\mu$m and a D90 value below 200 $\mu$m; and wherein the particle size distribution is characterized by a span of 2.0 or lower, and wherein particle size determinations are performed using a Helos Sympatec Particle Size Distribution analyzer, as further disclosed in Example 1 and wherein the span is defined as Span = (D90 - D10)/D50.

BRIEF DESCRIPTION OF THE DRAWINGS

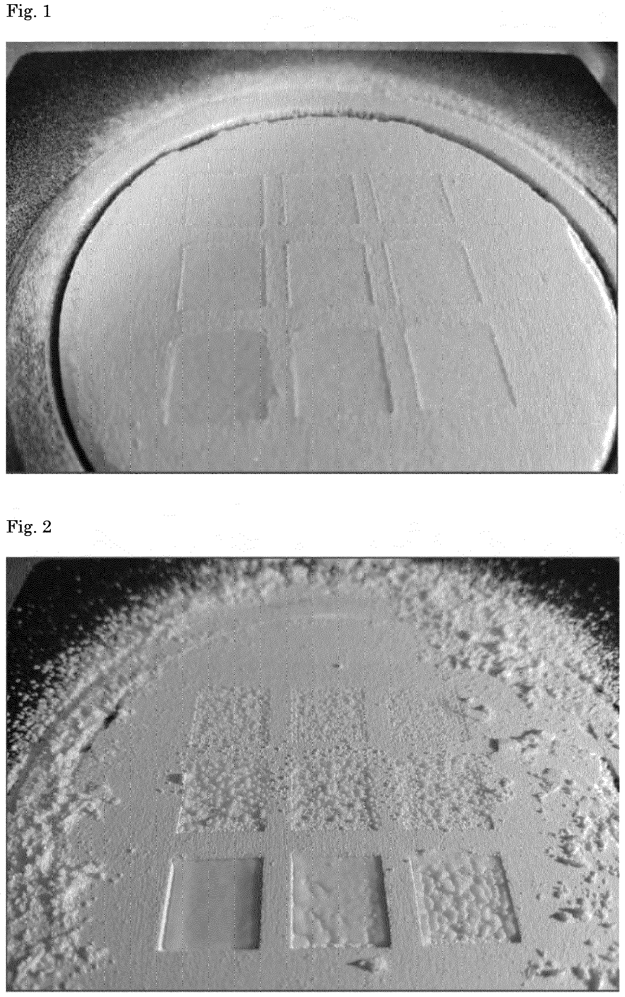

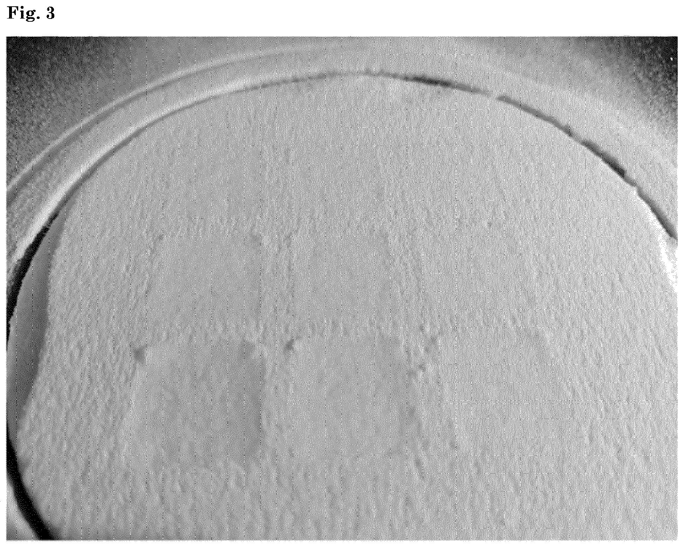

Figures 1, 2 and 3 show photographs of printed squares with lactose particles in accordance with the invention (Fig. 1) and with lactose particles not in accordance with the invention (Fig. 2, 3).

DETAILED DESCRIPTION

The invention is based on the finding that particle size distribution can affect the suitability of lactose particles in 3D printing. Particularly, the inventors have found that a relatively low presence of fines allows to provide suitable wettability by a printing liquid, Thereby, preferably, an improved consolidation and reduced bleeding of the printing liquid can be obtained. Consolidation refers to the extent to which the particles are kept together. Bleeding refers to the less desirable phenomenon that printing liquid applied on one layer of a powder bed, flows through to a lower layer of powder bed. This problem is typical for 3D printing, in view of the layer by layer nature of the printing process.

The desired size distribution is characterized by the requirement that the parameter D10 is at least 6 $\mu$m and a D90 value below 200 $\mu$m; and wherein the particle size distribution is characterized by a span of 2.0 or lower, and wherein particle size determinations are performed using a Helos Sympatec Particle Size Distribution analyzer, as further disclosed in Example 1 and wherein the span is defined as Span = (D90 - D10)/ D50.

The most commonly used metrics when describing particle size distributions are D-Values (D10, D50 & D90) which are the intercepts for 10%, 50% and 90% of the cumulative volume based fractions. D-values can be thought of as the diameter of the sphere which divides a sample's volume into a specified percentage when the particles are arranged on an ascending volume basis. Thus, the D10 is the diameter at which 10% of the sample's volume is comprised of particles with a diameter less than this value. Accordingly, the lactose particles selected for use in the present invention are such that 10% of the volume comprises particles having a diameter less than at least 6 $\mu$m. Accordingly, 90% of the volume comprises particles having a diameter, as determined by the aforementioned Laser diffraction method, of 6 $\mu$m or higher. In the field of lactose powders, this reflects a relatively large particle size, and at any rate a low presence of fines. D-values are also known as "d" or "x" values (i.e., D10 corresponds to x10). In order to optimize the printed layered structure, the D90 value is below 200 $\mu$m , more preferably below 150 $\mu$m.

The lactose particles used in the present invention preferably have a D10 value, as measured by the aforementioned method using a Helos Sympatec Particle Size Distribution analyzer, of at least 6 $\mu$m, such as at least 25 $\mu$m, such as at least 35 $\mu$m. Preferably, the D10 value is at most 60 $\mu$m, such as at most 50 $\mu$m. Preferred ranges for the D10 values are 10 $\mu$m to 25 $\mu$m, alternatively 25 $\mu$m to 35 $\mu$m, alternatively and 35 $\mu$ to 50 $\mu$m.

In an embodiment of the invention, the particle size distribution of the lactose particles used in the invention is characterized by the D50 value. It was found that the D50 value is suitably greater than 40 $\mu$m, such as greater than 50 $\mu$m, preferably greater than 65 $\mu$m.

The lactose particles used in the present invention preferably have a suitable flowability such as characterized by their Hausner ratio. The Hausner ratio is calculated by the formula

$$H = \frac{\rho_T}{\rho_B}$$

Therein $\rho_B$ is the poured bulk density of the powder, and $\rho_T$ is the tapped bulk density of the powder, as determined in accordance with the Examples.

The lactose particles used herein preferably have Hausner ratio of below 1.50, such as at most 1.41, such as below 1.3, such as below 1.2. The Hausner ratio is generally greater than 1.10.

Alternatively, the flowability of the present lactose powders is characterized by Carr's index, reflecting the compressibility of a powder.

The Carr index is calculated by the formula:

$$C = 100 \frac{V_T - V_B}{V_T}$$

Therein $V_B$ refers to the volume that a given mass of powder would occupy if allowed to settle freely. $V_T$ refers to the volume that the same mass of powder would occupy after tapping down. Carr's index can also expressed with reference to the above $\rho_B$ and $\rho_T$, as:

$$C = 100(1 - \rho_B/\rho_T)$$

The lactose particles used herein preferably have a Carr's index of below 30, preferably below 20.

An additional parameter to show the width of a particle size distribution is the span. The span of a volume-based size distribution is defined as

$$\mathrm{Span} = (\mathrm{D}90 - \mathrm{D}10)/\mathrm{D}50$$

and gives an indication of how far the 10 percent and 90 percent points are apart, normalized with the midpoint.

The lactose particles used in the present invention have a particle size distribution characterized by a relatively narrow span of 2.0 or lower, such as 1.0 to 2.0.

Preferred lactose particles, indicated with their tradename, can be listed as follows:

| | | Hausner | Carr's (%) | D10 | D50 | D90 | span |
|---|---|---|---|---|---|---|---|
| 1 | Respitose SV003 | 1.259 | 20.6 | 35.7 | 65.5 | 103.1 | 1.0 |
| 2 | Lactohale 206 | 1.191 | 16.0 | 36.7 | 87.5 | 161.7 | 1.4 |
| 3 | Respitose SV010 | 1.145 | 12.7 | 49.4 | 109.0 | 177.0 | 1.2 |
| 4 | Pharmatose 125M | 1.214 | 17.6 | 25.8 | 74.8 | 138.3 | 1.5 |
| 5 | Lactohale LH200 | 1.404 | 28.8 | 11.1 | 76.5 | 161.2 | 2.0 |

The lactose particles can be of generally any type, provided that the D10, D90 and span requirement of the invention is satisfied. The lactose can thus be alpha-lactose, beta-lactose, or a mixture thereof. The lactose can be crystalline lactose, amorphous lactose, or a mixture thereof. The lactose can be anhydrous lactose, a hydrate, such as alpha lactose monohydrate or a mixture thereof. The lactose can be sieved lactose, spray dried lactose, or a mixture thereof. Various mixtures of types of lactose and processing techniques to obtain particles, can be used. Preferably the lactose particles comprise or consist of alpha lactose monohydrate, most preferably non-spray-dried alpha lactose monohydrate.

In traditional solid pharmaceutical formulations, such as capsules and notably tablets, a regular combination of excipients is present. In some instances, tablets are made by directly compressing suitable powders. Frequently, tablets are made by first rendering powdered starting materials into a granulate, and then compressing the granulate. Granules can also be used to fill capsules. Some excipients have a function in the resulting formulation, e.g. stabilizers or colorants. Some excipients have a function in the processing of powders into granules.. Some excipients have a function as a direct compression aid. Excipients can also have combined functions.

Generally, the excipients in a pharmaceutical formulation comprise one or more of the following: a filler or diluent, to make the bulk of the formulation; a disintegrant to facilitate release and dissolution of a drug substance from the formulation; a binder to hold the components together and provide the formulation with sufficient mechanical strength; a lubricant as a processing aid to prevent granules from adhering to granulation and/or tabletting equipment; a glidant as a processing aid to improve flowability of powder from which granules or direct compression tablets are made. Other excipients can include further processing aids, such as wetting agents (surfactants), components that are desired depending on the type of drug and the manner in which it is to be released (e.g., adsorbents, buffers, antioxidants, chelating agents, dissolution enhancers, dissolution retardants), and agents that serve the palatability and patient-acceptance of the formulation (e.g., colorants, flavours, sweeteners).

At a level of formulation technology, 3D printing has requirements and possibilities different from the foregoing conventional formulation technology. As a result, conventional excipients, notably bulking agents (fillers, diluents) and the accompanying binders and disintegrants, and lubricants, are not necessarily suitable for 3D printing.

The present invention is specifically related to lactose as a bulking agent. An advantage of lactose is its widespread acceptability as a pharmaceutical excipient. The present invention allows producing pharmaceutical formulations by 3D printing, particularly using inkjet printing (i.e., known in the field as binder jetting), yet with the well-accepted pharmaceutical excipient lactose.

It will be understood that the present invention is directed to formulation technology, and is not drug-specific. I.e., it can be used with any drug that can be comprised in a solution or suspension or paste from which droplets can be produced by a jetting device, or with any drug substance that can suitably be blended into a powder bed. The technology to take up drug substances into 3D printed formulations is familiar to the skilled person. Generally, a drug is dissolved in a suitable solvent to form a solution. This solution can be mixed with other excipients to form a homogenous paste. This prepared paste is then fed into the 3D printer and sprayed in small droplets onto a powder bed for creating the drug product of the desired shape and volume. This product generally is considered a tablet, but it can be a solid pharmaceutical formulation of largely any desired shape, by virtue of the 3D printing process.

It will be understood that a pharmaceutical formulation can also be made without a drug. This is important, e.g., for clinical trials in which drug-loaded formulations are tested against placebo formulations. Generally, it is desired that the drug-loaded formulations and placebo formulations from appearance cannot be distinguished from each other.

As desired, pharmaceutical formulations produced by 3D printing can comprise one or more of conventional types of excipients such as referred to above. This can be any excipient suitable to be taken up either in a jetting liquid or in a powder bed. It will be understood that this particularly holds for excipients that are not specific for the formulation technology used. This refers, e.g., to excipients that depend on the type of drug, the desired release characteristics, and/or on the desired appearance and taste of the formulation.

The formulations of the present invention are produced by binderjetting on a powder bed, in a repeated fashion as explained above. The powder bed comprises lactose having the particle size characteristics as explained above. Thereby, in an embodiment, the powder bed consists essentially of said lactose particles, with at most minor quantities of other excipients present in the powder bed. This generally refers to at most 10 wt.% of such other excipients, such as less than 5 wt.%, such as less than 2 wt.%. In another embodiment, the powder bed consists of said lactose particles.

The liquid sprayed onto the powder bed is generally termed a "binder liquid." This does not imply that it solely comprises components that act as a binder. In fact, the liquid itself can be a binder, e.g., water or ethanol, or another suitable organic solvent. Suitable solvents are typically those solvents that are recognized as being pharmaceutically acceptable. These solvents are familiar to the skilled person, with preferred solvents being according to ICH Class 3 as determined under the 2017 FDA recommendations Q3C on impurities and residual solvents. Preferred solvents include water, ethanol and isopropanol. In the event that a drug is added to the formulation via the liquid, said liquid will generally comprise a solution or dispersion of a drug substance. The bulk of the liquid is preferably selected for its ability to wet lactose particles, e.g. water, or an alcohol such as ethanol or isopropanol. Preferably, the liquid is aqueous and can be a mixture of water and ethanol, e.g. in a volume ratio of 5/95 to 95/5, such as 20/80 to 80/20, such as 60/40 to 40/60. More preferably, the liquid is water or water with 5% ethanol. Suitable binders to be dissolved or dispersed into said liquid are: pregelatinized or partly pregelatinized starch, preferably pregelatinized potato starch (such as sold under the name Prejel) and/or partly pregelatinized maize starch; starch derivatives such as crosslinked starch or carboxymethyl starch, cellulose derivatives such as hydroxypropyl cellulose (HPC) or hydroxypropyl methyl cellulose (HPMC), hydrophilic polymers such as polyvinyl pyrrolidone (PVP) or polyvinyl alcohol (PVA). The binder and the lactose can also be present in a single, co-processed particle. The latter is to be understood as a lactose particle in accordance with the invention. Such co-processed particle will thus have a D10 of at least 6 $\mu$m and a D90 value below 200 $\mu$m; and the particle size distribution characterized by a span of 2.0 or lower, the particle size determined using a Helos Sympatec Particle Size Distribution analyzer, as further disclosed in Example 1 and the span as Span = (D90 - D10)/D50, as defined above.

In a preferred embodiment, a binder is not only added via a jetting liquid, but is also present in the powder bed. Hereby the powder bed comprises the aforementioned lactose particles and particles of at least one binder. The binder particles will generally be present in a range up to 35% mass%, such as up to 25 mass%, preferably of from 10 mass% to 20 mass%. In an embodiment, the powder bed consists essentially of said lactose particles and said binder particles, with at most minor quantities of other excipients present in the powder bed, as mentioned above.

Suitable binder particles generally can be blended with the lactose particles such that the resulting powder bed retains a suitable flowability. It is preferred that the binder particles are homogeneously distributed over the bulk of lactose particles. This can be achieved in various ways, and the skilled person will be able to assess the resulting flowability of the blend by standard methods. By way of non-limiting further guidance, binder particles can be provided that have a flowability comparable to that of the lactose particles. E.g., in that event the flowability parameters such as Carr's index values of the binder particles preferably deviate from the same parameters of the lactose particles by at most 25%, such as at most 15%, preferably at most 10%. An advantage of the lactose particles selected according to the invention, is that their flowability properties generally allow to compensate for poor flowability of binder particles. Accordingly, the invention allows the presence of relatively large amounts of binder particles that do no necessarily have a flowability similar to that of the lactose.

Preferred binder particles comprise polyvinyl pyrrolidone (PVP), pregelatinized starch such as Prejel (potato starch), hydroxy propyl cellulose (HPC), hydroxy propyl methyl cellulose (HPMC).

Another component of the powder from which powder beds are provided, can be a drug substance. These will generally be solid substances of any form, crystalline, solvates, amorphous, that can be blended with the lactose particles. Here, too, the selected lactose particles have the advantages of allowing to be blended with particles of different flowability.

In an embodiment, the powder bed will have the same composition in each run of jetting liquid onto it. In another embodiment, the powder beds as applied in or more of the runs, can be different. E.g., it is conceivable to generate central layers having, e.g., more binder particles than layers that end op on the outside of the 3D printed solid dosage form. Similarly, the jetting liquid can be the same in each run, or one or more of the runs can be carried out with a different jetting liquid.

In another aspect, the invention is a process of preparing a solid pharmaceutical formulation by three-dimensional (3D) printing. The process comprises providing a first layer of a powder, depositing a pattern of binder liquid onto said first layer so as to provide a bound layer of powder. Subsequently, usually by lowering the first layer, a second layer of the powder is applied onto the first layer. On this second layer again a pattern of binder liquid is deposited, so as to provide a set of two bound layers of powder. The foregoing steps are continued, i.e., by applying further layers of powder each followed by further deposition of binder liquid, so as to provide a printed three-dimensional solid pharmaceutical formulation. The patterns in each layer can be the same or different, depending on the desired three-dimensional shape of the final product. In accordance with the invention, the powder comprises lactose particles having a particle size distribution characterized by a D10 of at least 6 $\mu$m and a D90 value below 200 $\mu$m; and wherein the particle size distribution is characterized by a span of 2.0 or lower, and wherein particle size determinations are performed using a Helos Sympatec Particle Size Distribution analyzer, as further disclosed in Example 1 and wherein the span is defined as Span = (D90 - D10)/D50, as substantially described above in all its embodiments.

The 3D printing process can be conducted using equipment and settings with which the skilled person is familiar.

Suitable printers are, e.g. Projet 460 3D printer, Sideswipe printer or Rapix3D 2.1.21.0 Forwiss.

In another aspect, the invention provides a kit for generating a powder bed suitable in the production of pharmaceutical formulations by 3D printing. The kit comprise lactose particles and binder particles, wherein the lactose particles have a particle size distribution characterized by a D10 of at least 6 $\mu$m and a D90 value below 200 $\mu$m; and wherein the particle size distribution is characterized by a span of 2.0 or lower, and wherein particle size determinations are performed using a Helos Sympatec Particle Size Distribution analyzer, as further disclosed in Example 1 and wherein the span is defined as Span = (D90 - D10)/D50.

The binder particles are as discussed above, and preferably are selected from the group consisting of pregelatinized starch, such as pregelatinized potato starch, HPC, HPMC, PVP, and PVA. In a preferred embodiment, the particles are a co-processed excipient, comprising lactose and binder.

In sum, the invention is the 3D printing of pharmaceutical formulations, such as tablets. Thereby a binder liquid is deposited dropwise on a layer of powder, with these steps being repeated, in one or more patterns, in order to generate a solid drug product. In accordance with the invention, the powder comprises lactose particles having a particle size distribution characterized by a D10 of at least 6 $\mu$m and a D90 value below 200 $\mu$m; and wherein the particle size distribution is characterized by a span of 2.0 or lower, and wherein particle size determinations are performed using a Helos Sympatec Particle Size Distribution analyzer, as further disclosed in Example 1 and wherein the span is defined as Span = (D90 - D10)/ D50.

The invention will be further explained with reference to the following non-limiting examples, and comparative examples.

Example 1

Lactose characterization

All ingredients are available from DFE Pharma GmbH, Germany, except Povidon K30, which was obtained from Duchefa Farma. All lactose grades used for 3D printing were measured on particle size and bulk and tapped density.

Method to determine particle size:

The particle sizing experiments were conducted using a Helos laser diffraction unit in conjunction with Windox 5 software (5.10.0.04), both from Sympatec GmbH (Clausthal-Zellerfield, Germany). The optical bench was equipped with R5 Fourier lens for focusing the diffraction patterns of equally sized particles to the same position on the array of detector rings. This lens covers particle size ranges between 4.5 and 850 $\mu$m. The Windox software is analyzing according the FREE mode for calculating the particle size distributions from the raw scattering data. The lactose powders were dispersed dry using the Rodos dry powder disperser in conjunction with the Vibri dry powder feeder (Sympatec). Before the measurements, the vacuum was switched on and the background scattering was recorded for 10 seconds. The sample was then introduced to the measurement zone with a steady flow rate. This was obtained by adjusting the gap between the funnel and the powder feeder tray to 2 mm. The vibration feed rate was set at 85% and data collection was started at a signal strength at Channel 12 >0.2% and data collection was maintained between 0.5% and 90% on channel 12. A pressure titration was performed to assess the disperser pressure at which the particles were fully dispersed and no attrition took place. On the basis of the results, a disperser pressure of 1.5 bar was selected. All Sympatec setting are summarized in Table 1 below.

The span was calculated by the formula: span= (D90-D10)/D50 The bulk and tapped density were measured by putting 100g weighted sample in a cylinder. The cylinder was put on the Poured and Tapped Density Meter Stav 2003 and repetitively 2500 taps were provided and the volume was noted. The Hausner ratio was calculated by the formula: Hausner ratio=tapped density/bulk density. The poured bulk density is indicated below as PBD and the tapped bulk density as TBD.

Table 1: Helos particle size settings used for the analysis

| Parameter | Settings / Description |
|---|---|
| Type | H3575 |
| Windox version | Windox 5.10.0.4 |
| Calculation model | Free mode |
| Sympatec Lens | R5 Fourier lens |
| Lens range | 4.5 $\mu$m to 850 $\mu$m |
| *Dispersion settings* | |
| Pressure | 1.5 bar |
| Vibration feed rate | 85.00% |
| Funnel Height | 2.00 mm |
| *Trigger Conditions* | |
| Time base | 100.00 ms |
| Start | Channel 12 $\geq$ 0.2% |
| Valid | 0.5% $\leq$ Channel 12 $\leq$ 90% |
| Stop | 5s Channel 12 $\leq$0.2% OR 30s real time |

Table 2: Powder characteristics

| | | PBD g/ml | TBD g/ml | Hausner | D10 ($\mu$m) | D50 ($\mu$m) | D90 ($\mu$m) | span |
|---|---|---|---|---|---|---|---|---|
| 1 | Respitose SV003 | 0.74 | 0.93 | 1.26 | 35.7 | 65.5 | 103.1 | 1.0 |
| 2 | Lactohale 206 | 0.76 | 0.91 | 1.19 | 36.7 | 87.5 | 161.7 | 1.4 |
| 3 | Pharmatose 125M | 0.74 | 0.89 | 1.21 | 25.8 | 74.8 | 138.3 | 1.5 |
| 4 | Lactohale LH200 | 0.69 | 0.96 | 1.40 | 11.1 | 76.5 | 161.2 | 2.0 |
| 5 | Lactochem Regular Powder | 0.66 | 0.94 | 1.42 | 5.6 | 45.9 | 139.5 | 2.9 |
| 6 | Pharmatose 200M NZ | 0.56 | 0.88 | 1.58 | 4.0 | 30.5 | 102.5 | 3.2 |
| 7 | Pharmatose 350M NZ | 0.52 | 0.82 | 1.58 | 3.8 | 26.9 | 77.1 | 2.7 |
| 8 | Supertab 11 SD | 0.61 | 0.75 | 1.24 | 39.4 | 110.1 | 203.2 | 1.5 |

(continued)

|  |  | PBD g/ml | TBD g/ml | Hausner | D10 (μm) | D50 (μm) | D90 (μm) | span |
|---|---|---|---|---|---|---|---|---|
| 9 | Sieved anhydrous lactose | 0.68 | 0.91 | 1.35 | 6.7 | 70.2 | 177.3 | 2.4 |

## Example 2

### Printing squares

In order to evaluate if a formulation is suitable for tablet printing a square can be printed (one layer of powder) and evaluated on wetting, consolidation and bleeding. The square printing was performed as follows.

The formulation as given in the tables were mixed on a roller bank until it was homogeneous. The formulations were printed with a Sideswipe printer making use of Pronterface software. The powder blend was manually spread on the printing desk with the help of a hand sieve (700 micron) and automatically rolled out with a roller on a printer. A water/ethanol (95/5 vol./vol.) mixture was sprayed on the powder bed with a 0.165 mm printhead (the lee company) at 0.175 bar, into 9 squares of 1.4 cm$^2$. For each square a different line spacing was used (as indicated in Table 3), resulting in 9 different wetted squares.

Table 3: Print settings for printing squares in a single layer of powder

| Sample | Line spacing (mm) | Droplets/mm |
|---|---|---|
| 1 | 0.2 | 5 |
| 2 | 0.25 | 4 |
| 3 | 0.3 | 3.3 |
| 4 | 0.35 | 2.8 |
| 5 | 0.4 | 2.5 |
| 6 | 0.45 | 2.2 |
| 7 | 0.5 | 2 |
| 8 | 0.55 | 1.9 |
| 9 | 0.6 | 1.6 |

Table 4: Print formulation and evaluation.

| (A mixture (vol %) of 5% Ethanol and 95% water was used as printing liquid) | |
|---|---|
| Excipients (wt.%) | Evaluation |
| 10% Prejel and 90% Lactohale 206 | Suitable |
| 10% Prejel and 90% Pharmatose 125M | Suitable |
| 10% Prejel and 90% Lactohale LH200 | Suitable |
| 10% Prejel and 90% Lactochem regular powder | Not suitable |
| 10% Prejel and 90% Pharmatose 350M NZ | Not suitable |

In Figures 1 and 2, photographs are given of squares printed with 95% water/5% ethanol with the 9 different line spacings as indicated in Table 3. Fig. 1 shows the result as obtained with the combination of 10% Prejel and 90% Lactohale 206 (according to the invention). Fig. 2 shows the result obtained with the combination of 10% Prejel and 90% Pharmatose 350M. It can clearly be seen that the wetting of the Pharmatose 350M was not suitable, resulting in a rough surface which is not suitable for applying another layer. On the other hand. Lactohale 206 resulted in a very smooth surface on which next layers can be deposited to print a tablet.

Table 5: Print formulation and evaluation.

| (A mixture (vol %) 5% Ethanol and 95% water was used as printing liquid) | |
| --- | --- |
| Excipients (wt %) | Evaluation |
| 20% Povidone k30 and 80% Lactohale 206 | Suitable |
| 20% Prejel and 80% Lactohale 206 | Suitable |
| 20% Povidone k30 and 80% Respitose SV003 | Suitable |
| 20% Povidone k30 and 80% Pharmatose 200M NZ | Not suitable |

Table 6: Print formulation and evaluation.

| (100% Ethanol was used as printing liquid) | |
| --- | --- |
| Excipients (wt %) | Evaluation |
| 10% Povidone k30 and 90% Lactohale 206 | Suitable |

Table 7: Print formulation and evaluation.

| (A mixture (vol %) 5% Ethanol and 95% water was used as printing liquid) | |
| --- | --- |
| Excipients | Evaluation |
| 10% Prejel and 90% Supertab 11SD | Suitable* |
| 10% Prejel and 90% sieved anhydrous powder | Suitable* |
| *Some bleeding observed in comparison with lactose monohydrate. | |

For the sample Prejel and Supertab 11SD (a spray-dried lactose), the squares had a wet appearance (Fig. 3) After drying, the squares were curved, not flat anymore. The consolidation and thickness are not optimal, and probably tablet printing will be difficult.

**Example 3**

Printing tablets

The formulation as given in the Table 8 were mixed on a roller bank until it was a homogeneous mixture. The formulations were printed with a Sideswipe printer making use of Pronterface software.

The powder blend was manually spread on the printing desk with the help of a hand sieve (700 micron) and automatically rolled out with a roller on a printer. A water/ethanol (95/5 vol./vol.) mixture was sprayed on the powder bed with a 0.165 mm printhead (the lee company) at 0.175 bar, with a line spacing of 0.45 mm and with 2.22 drops/mm in the shape of 9mm circle. The powder deposition and solution spraying was repeated 7 times in order to create a flat tablet with a diameter of 9 mm and a height of 3 mm. Tablets were removed from the powder bed and dried in an oven overnight at 50°C. Suitable tablets were obtained with dimensions and masses as indicated in Table 9.

Table 8: Tablet formulations (wt%)

| | 1 | 2 | 3 | 4 |
| --- | --- | --- | --- | --- |
| Lactohale 206 | 80 | - | 80 | 90 |
| Respitose SV003 | - | 80 | - | - |
| Prejel | - | - | - | - |
| Povidone K30 | 20 | 20 | 20 | 10 |

Table 9: Tablet mass and dimensions

| | Mass (mg) | Diameter (mm) | Height (mm) |
| --- | --- | --- | --- |
| 80% Lactohale 206/20% PVP | 140.9 | 9.35 | 3.57 |

(continued)

| | Mass (mg) | Diameter (mm) | Height (mm) |
|---|---|---|---|
| 80% Respitose SV003/20% PVP | 119.8 | 9.35 | 3.22 |
| 80% Lactohale 206/20% Prejel | 126.9 | 9.24 | 3.24 |
| 90% Lactohale 206/10% Prejel | 148.8 | 9.4 | 3.56 |

Example 4.

Printing tablets and measurement of tablet properties.

Milled and classified lactose monohydrate with a D10 above 20 $\mu$m (90 wt%) and fully pregelatinized starch (10 wt%) were mixed on a roller bank until it was a homogeneous mixture. The formulations were printed with a PBP Next printer with a Lee valve INKA2476210H (0.178 mm). The powder blend was automatically dispersed from a hopper onto the powder platform and automatically rolled out with a roller on the printer. A water/ethanol (95/5 vol./vol.) mixture was sprayed on the powder bed with a line spacing of 0.43 mm and with 2.22 drops/mm in a line wise direction. The powder deposition and solution spraying was repeated 7 times in order to create a flat tablet with a diameter of 9 mm and a height of 2.8 mm. Tablets were subsequently removed from the powder bed and dried in an oven overnight at 50°C.

The tablets were found to have an acceptable hardness of 61N, measured with an automated tablet tester (Sotax HT100) at constant speed of 120 mm/min (2mm/s). Furthermore, the tablets were found to have a fast disintegration time of 52 seconds, measured in demineralized water of 37°C with a disintegration tester (ex Erweka GmbH) equipped with sinkers. These properties were considered very suitable for tablets.

## Claims

1. The use of lactose particles in the production of pharmaceutical formulations by three-dimensional (3D) printing, said printing comprising the repeated deposition of liquid drops onto superimposed layers of a powder bed, wherein the powder bed comprises lactose particles having a particle size distribution **characterized by** a D10 of at least 6 $\mu$m and a D90 value below 200 $\mu$m; and wherein the particle size distribution is **characterized by** a span of 2.0 or lower, and wherein particle size determinations are performed using a Helos Sympatec Particle Size Distribution analyzer, as further disclosed in Example 1 and wherein the span is defined as Span = (D90 - D10)/D50.

2. A use according to claim 1, wherein the D10 is at least 10 $\mu$m, preferably at least 25 $\mu$m.

3. A use according to any one of the preceding claims, wherein the D10 values is in a range of from 10 $\mu$m to 25 $\mu$m.

4. A use according to any one of claims 1 to 3, wherein the D10 value is in a range of from 25 $\mu$m to 35 $\mu$m.

5. A use according to any one of claims 1 to 3, wherein the D10 value is in a range of from and 35 $\mu$m to 50 $\mu$m.

6. A use according to any one of the preceding claims, wherein the lactose particles have a bulk density **characterized by** either or both of a Hausner ratio of between 1.10 and 1.50 or a Carr's index of below 30.

7. A use according to claim 1, wherein the powder bed further comprises particles of a binder, preferably selected from the group consisting of pregelatinized starch, starch derivatives, cellulose derivatives, hydrophilic polymers, and combinations thereof.

8. A use according to claim 7, wherein the binder is selected from the group consisting of pregelatinized potato starch, hydroxypropyl cellulose, hydroxypropyl methyl cellulose, polyvinyl pyrrolidone, polyvinyl alcohol, and mixtures thereof.

9. A use according to any one of the preceding claims, wherein the liquid drops, the powder bed, or both, comprise a drug substance.

10. A kit for generating a powder bed suitable in the production of pharmaceutical formulations by 3D printing, the kit comprising lactose particles and binder particles, wherein the lactose particles have a particle size distribution reflected by a D10 of at least 6 $\mu$m and a D90 value below 200 $\mu$m; and wherein the particle size distribution is **characterized by** a span of 2.0 or lower, wherein particle size determinations are performed using a Helos Sympatec Particle Size Distribution analyzer, as further disclosed in Example 1 and wherein the span is defined as Span = (D90 - D10)/D50.

11. A kit according to claim 10, wherein the lactose particles are as defined in any one of the claims 2 to 6.

12. A kit according to claim 10 or 11, wherein the binder particles are as defined in claim 7 or 8.

13. A process of preparing a solid pharmaceutical formulation by three-dimensional (3D) printing, comprising providing a first layer of a powder, depositing a pattern of binder liquid onto said first layer so as to provide a bound layer of powder, applying a second layer of the powder onto the first layer, depositing a pattern of binder liquid onto said second layer so as to provide a set of two bound layers of powder, continuing to provide further layers of powder each followed by further deposition of binder liquid, so as to provide a printed three-dimensional solid pharmaceutical formulation, wherein the powder comprises lactose particles having a particle size distribution reflected by a D10 of at least 6 $\mu$m and a D90 value below 200 $\mu$m; and wherein the particle size distribution is **characterized by** a span of 2.0 or lower, wherein particle size determinations are performed using a Helos Sympatec Particle Size Distribution analyzer, as further disclosed in Example 1 and wherein the span is defined as Span = (D90 - D10)/D50.

14. A process according to claim 13, wherein the lactose particles are as defined in any one of the claims 2 to 6 and wherein preferably one or more of the layers of powder further comprise particles of a binder as defined in claim 7 or 8.


**Patentansprüche**

1. Verwendung von Laktosepartikeln bei der Herstellung von pharmazeutischen Formulierungen durch dreidimensionales (3D) Drucken, wobei das Drucken das wiederholte Ablagern von Flüssigkeitstropfen auf übereinanderliegenden Schichten eines Pulverbetts umfasst, wobei das Pulverbett Laktosepartikel mit einer Partikelgrößenverteilung umfasst, die durch einen D10-Wert von mindestens 6 $\mu$m und einen D90-Wert unter 200 $\mu$m gekennzeichnet ist; und wobei die Partikelgrößenverteilung durch eine Spanne von 2,0 oder weniger gekennzeichnet ist, und wobei Partikelgrößenbestimmungen unter Verwendung eines Helos Sympatec Partikelgrößenverteilungsanalysators durchgeführt werden, wie weiterhin in Beispiel 1 offenbart, und wobei die Spanne als Spanne = (D90 - D10)/D50 definiert ist.

2. Verwendung nach Anspruch 1, wobei der D10-Wert mindestens 10 $\mu$m, vorzugsweise mindestens 25 $\mu$m ist.

3. Verwendung nach einem der vorhergehenden Ansprüche, wobei die D10-Werte in einem Bereich von 10 $\mu$m bis 25 $\mu$m sind.

4. Verwendung nach einem der Ansprüche 1 bis 3, wobei der D10-Wert in einem Bereich von 25 $\mu$m bis 35 $\mu$m ist.

5. Verwendung nach einem der Ansprüche 1 bis 3, wobei der D10-Wert in einem Bereich von 35 $\mu$m bis 50 $\mu$m ist.

6. Verwendung nach einem der vorhergehenden Ansprüche, wobei die Laktosepartikel eine Schüttdichte aufweisen, die entweder durch ein Hausner-Verhältnis zwischen 1,10 und 1,50 oder durch einen Carr-Index unter 30 oder durch beides gekennzeichnet ist.

7. Verwendung nach Anspruch 1, wobei das Pulverbett weiterhin Partikel eines Bindemittels umfasst, das vorzugsweise aus der Gruppe ausgewählt ist, bestehend aus vorverkleisterter Stärke, Stärkederivaten, Cellulosederivaten, hydrophilen Polymeren und ihren Kombinationen.

8. Verwendung nach Anspruch 7, wobei das Bindemittel aus der Gruppe ausgewählt ist, bestehend aus vorverkleisterter Kartoffelstärke, Hydroxypropylcellulose, Hydroxypropylmethylcellulose, Polyvinylpyrrolidon, Polyvinylalkohol und ihren Mischungen.

9. Verwendung nach einem der vorhergehenden Ansprüche, wobei die Flüssigkeitstropfen, das Pulverbett oder beide eine Arzneimittelsubstanz umfassen.

10. Kit zum Erzeugen eines Pulverbetts, das für die Herstellung von pharmazeutischen Formulierungen durch 3D-Drucken geeignet ist, wobei das Kit Laktosepartikel und Bindemittelpartikel umfasst, wobei die Laktosepartikel eine Partikelgrößenverteilung aufweisen, die durch einen D10-Wert von mindestens 6 $\mu$m und einen D90-Wert unter 200 $\mu$m ausgedrückt wird, und wobei die Partikelgrößenverteilung durch eine Spanne von 2,0 oder weniger gekennzeichnet ist, wobei Partikelgrößenbestimmungen unter Verwendung eines Helos Sympatec Partikelgrößenverteilungsanalysators durchgeführt werden, wie weiterhin in Beispiel 1 offenbart, und wobei die Spanne als Spanne = (D90 - D10)/D50 definiert ist.

11. Kit nach Anspruch 10, wobei die Laktosepartikel nach einem der Ansprüche 2 bis 6 definiert sind.

12. Kit nach Anspruch 10 oder 11, wobei die Bindemittelpartikel nach Anspruch 7 oder 8 definiert sind.

13. Verfahren zum Herstellen einer festen pharmazeutischen Formulierung durch dreidimensionales (3D) Drucken, umfassend Bereitstellen einer ersten Schicht eines Pulvers, Ablagern eines Musters von Bindemittelflüssigkeit auf der ersten Schicht, um eine gebundene Pulverschicht bereitzustellen, Aufbringen einer zweiten Schicht des Pulvers auf der ersten Schicht, Ablagern eines Musters von Bindemittelflüssigkeit auf der zweiten Schicht, um einen Satz von zwei gebundenen Pulverschichten bereitzustellen, Fortsetzen des Bereitstellens weiterer Pulverschichten, jeweils gefolgt von weiterem Ablagern von Bindemittelflüssigkeit, um eine gedruckte dreidimensionale feste pharmazeutische Formulierung bereitzustellen, wobei das Pulver Laktosepartikel mit einer Partikelgrößenverteilung umfasst, die durch einen D10-Wert von mindestens 6 $\mu$m und einen D90-Wert unter 200 $\mu$m ausgedrückt wird; und wobei die Partikelgrößenverteilung durch eine Spanne von 2,0 oder weniger gekennzeichnet ist, wobei Partikelgrößenbestimmungen unter Verwendung eines Helos Sympatec Partikelgrößenverteilungsanalysators durchgeführt werden, wie weiterhin in Beispiel 1 offenbart, und wobei die Spanne als Spanne = (D90 - D10)/D50 definiert ist.

14. Verfahren nach Anspruch 13, wobei die Laktosepartikel nach einem der Ansprüche 2 bis 6 definiert sind und wobei vorzugsweise mindestens eine der Pulverschichten weiterhin Partikel eines Bindemittels nach Anspruch 7 oder 8 umfasst.

## Revendications

1. Utilisation de particules de lactose dans la production de formulations pharmaceutiques par impression tridimensionnelle (3D), ladite impression comprenant le dépôt répété de gouttes liquides sur des couches superposées d'un lit de poudre, le lit de poudre comprenant des particules de lactose ayant une distribution granulométrique **caractérisée par** un D10 d'au moins 6 $\mu$m et une valeur D90 inférieure à 200 $\mu$m ; et la distribution granulométrique étant **caractérisée par** un span de 2,0 ou inférieur, et
des déterminations de taille de particules étant effectuées en utilisant un analyseur de distribution granulométrique Helos Sympatec, tel que divulgué dans l'Exemple 1 et le span étant défini comme Span = (D90-D10)/D50.

2. Utilisation selon la revendication 1, le D10 étant d'au moins 10 $\mu$m, de préférence d'au moins 25 $\mu$m.

3. Utilisation selon l'une quelconque des revendications précédentes, la valeur D10 étant dans une plage de 10 $\mu$m à 25 $\mu$m.

4. Utilisation selon l'une quelconque des revendications 1 à 3, la valeur D10 étant dans une plage de 25 $\mu$m à 35 $\mu$m.

5. Utilisation selon l'une quelconque des revendications 1 à 3, la valeur D10 étant dans une plage de 35 $\mu$m à 50 $\mu$m.

6. Utilisation selon l'une quelconque des revendications précédentes, les particules de lactose ayant une densité apparente **caractérisée par** l'un ou l'autre ou les deux d'un rapport de Hausner entre 1,10 et 1,50 ou un indice de Carr inférieur à 30.

7. Utilisation selon la revendication 1, le lit de poudre comprenant en outre des particules d'un liant, de préférence sélectionné dans le groupe constitué d'amidon prégélatinisé, de dérivés d'amidon, de dérivés de cellulose, de polymères hydrophiles, et de combinaisons de ceux-ci.

8. Utilisation selon la revendication 7, le liant étant sélectionné dans le groupe constitué d'amidon de pomme de terre prégélatinisé, d'hydroxypropyl cellulose, d'hydroxypropyl méthyl cellulose, de polyvinyl pyrrolidone, d'alcool polyvinylique, et de mélanges de ceux-ci.

9. Utilisation selon l'une quelconque des revendications précédentes, les gouttes liquides, le lit de poudre, ou les deux, comprenant une substance médicamenteuse.

10. Kit pour générer un lit de poudre approprié dans la production de formulations pharmaceutiques par impression 3D, le kit comprenant des particules de lactose et des particules de liant, les particules de lactose ayant une distribution granulométrique reflétée par un D10 d'au moins 6 $\mu$m et une valeur D90 inférieure à 200 $\mu$m ; et la distribution granulométrique étant **caractérisée par** un span de 2,0 ou inférieur, des déterminations de taille de particules étant effectuées en utilisant un analyseur de distribution granulométrique Helos Sympatec, tel que divulgué dans l'Exemple 1 et le span étant défini comme Span = (D90-D10)/D50.

11. Kit selon la revendication 10, les particules de lactose étant telles que définies selon l'une quelconque des revendications 2 à 6.

12. Kit selon la revendication 10 ou 11, les particules de liant étant telles que définies dans la revendication 7 ou 8.

13. Procédé de préparation d'une formulation pharmaceutique solide par impression tridimensionnelle (3D), comprenant la fourniture d'une première couche de poudre, le dépôt d'un motif de liquide liant sur ladite première couche de manière à fournir une couche liée de poudre, l'application d'une seconde couche de poudre sur la première couche, le dépôt d'un motif de liquide liant sur ladite seconde couche de manière à fournir un ensemble de deux couches liées de poudre, la poursuite de la fourniture de couches supplémentaires de poudre chacune suivie par un dépôt supplémentaire de liquide liant, de manière à fournir une formulation pharmaceutique solide tridimensionnelle imprimée, la poudre comprenant des particules de lactose ayant une distribution granulométrique reflétée par un D10 d'au moins 6 $\mu$m et une valeur D90 inférieure à 200 $\mu$m ; et la distribution granulométrique étant **caractérisée par** un span de 2,0 ou inférieur, les déterminations de taille de particules étant effectuées en utilisant un analyseur de distribution granulométrique Helos Sympatec, tel que divulgué dans l'Exemple 1 et le span étant défini comme Span = (D90-D10)/D50.

14. Procédé selon la revendication 13, les particules de lactose étant telles que définies selon l'une quelconque des revendications 2 à 6 et de préférence une ou plusieurs des couches de poudre comprenant en outre des particules d'un liant tel que défini dans la revendication 7 ou 8.

Fig. 1

Fig. 2

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20020106412 A
- US 20110187015 A